# EUROPEAN PATENT APPLICATION

(11) **EP 3 318 316 A1**
(43) Date of publication of application: **09.05.2018**
(21) Application number: 17200394.9
(22) Date of filing: 07.11.2017
(51) Int. Cl.: B01D 53/86, B01J 8/02, B01D 53/88

(54) **METHANE CONVERTER AND METHOD OF CONVERTING METHANE EMISSIONS**

(30) Priority: 07.11.2016 US 201662418406 P
(71) Applicant: CCI Thermal Technologies Inc., Edmonton, Alberta T6B 3E1 (CA)
(72) Inventor: MALDONADO, Alejandro, Oakville, Ontario L6H 5R5 (CA); WERAN, Lorne, Edmonton, Alberta T6B 3E1 (CA); MAH, Kor Zheng, Oakville, Ontario L6H 5R5 (CA); SZYNKARCZUK, Jarek, Edmonton, Alberta T6B 3E1 (CA)
(74) Representative: Richardt Patentanwälte PartG mbB

(57) **Abstract**

A methane converter for converting methane into carbon dioxide and water, the converter comprising a gas feed for feeding methane into the converter, a mesh pad separator for receiving the methane from the gas feed and for separating methane gas from liquid, a drain connected to the separator to drain off the liquid, an air intake for receiving air, a tubular section extending upwardly from the air intake, a methane nozzle connected to the separator for receiving the methane gas from the separator and for discharging the methane gas inside the tubular section at a point downstream of the air intake, a catalyst bed within the tubular section for reacting the methane gas with oxygen in the air to form the carbon dioxide and the water, and an outlet of the tubular section for emitting the carbon dioxide and the water.

## Description

### TECHNICAL FIELD

The present invention relates to methods and systems for converting methane emissions from industrial processes.

### BACKGROUND

Methane emissions from industrial processes such as oil and gas plants are an environmental concern. Methane emissions may be due to venting and flaring. In addition, some methane emissions are fugitive emissions that come from valves, pumps, regulators, joints, flanges, meters or other equipment that leak gas.

Methane (CH₄) is 84% more potent as a greenhouse gas (GHG) than carbon dioxide. Controlling vented and fugitive methane emissions in the oil and gas industry is thus extremely important to prevent GHG-induced climate change.

Some attempts to date in the oil and gas industry to convert methane to less pernicious carbon dioxide have been only partially successful. The prior-art converters proposed to date have been unable achieve complete conversion of methane. Furthermore, the prior-art converters involve active mechanical switching. A passive converter capable of completely converting methane would thus be highly desirable.

### SUMMARY

The following presents a simplified summary of some aspects or embodiments of the invention in order to provide a basic understanding of the invention. This summary is not an extensive overview of the invention. It is not intended to identify key or critical elements of the invention or to delineate the scope of the invention. Its sole purpose is to present some embodiments of the invention in a simplified form as a prelude to the more detailed description that is presented later.

The present specification discloses a methane converter and a method of converting methane which is passive and more complete than the prior art. The converter and method may be used to convert methane to carbon dioxide that is less detrimental to the environment. In general, the converter and method have a catalyst bed disposed inside a tubular section. Methane gas from a separator is discharged into the tubular section upstream of an air intake to enable the methane gas and oxygen from the air to react in the catalyst bed to form carbon dioxide and water. The tubular section may be connected to, or part of, a stack that provides natural draft forces to displace the methane gas and air through the catalyst bed. An optional fan may be used to draw the methane and air through the catalyst bed.

One inventive aspect of the disclosure is a methane converter for converting methane into carbon dioxide and water. The converter includes a gas feed for feeding methane into the converter, a mesh pad separator for receiving the methane from the gas feed and for separating methane gas from liquid, a drain connected to the separator to drain off the liquid, an air intake for receiving air, a tubular section extending upwardly from the air intake, a methane nozzle connected to the separator for receiving the methane gas from the separator and for discharging the methane gas inside the tubular section at a point downstream of the air intake, a catalyst bed within the tubular section for reacting the methane gas with oxygen in the air to form the carbon dioxide and the water, and an outlet of the tubular section for emitting the carbon dioxide and the water.

Another inventive aspect of the disclosure is a method of converting methane into carbon dioxide and water by reacting the methane with oxygen contained in air drawn into the converter. The method (or process) entails various steps, acts or operations which may be performed in an order other than what is described below. Some of the steps, acts or operations may also be performed simultaneously or partially overlapping in time. In one embodiment, the method entails a step of feeding methane into the converter via a gas feed and separating methane gas from liquid using a mesh pad separator. The method also entails draining off the liquid from the separator. The method further entails receiving air into the converter via an air intake and flowing the air upwardly from the air intake through a tubular section. The method further involves discharging the methane gas received from the separator through a methane nozzle inside the tubular section at a point downstream of the air intake and reacting, in a catalyst bed within the tubular section, the methane gas with oxygen in the air to form the carbon dioxide and the water. The method further involves emitting the carbon dioxide and the water via an outlet of the tubular section.

### BRIEF DESCRIPTION OF THE DRAWINGS

These and other features of the disclosure will become more apparent from the description in which reference is made to the following appended drawings.
Figure 1 depicts a methane converter in accordance with a first embodiment of the present invention.
Figure 2 depicts a methane converter in accordance with a second embodiment of the present invention.
Figure 3 depicts a methane converter in accordance with a third embodiment of the present invention.
Figure 4 depicts a methane converter in accordance with a fourth embodiment of the present invention.
Figure 5 depicts an exemplary use of the converter in an oil facility.

### DETAILED DESCRIPTION OF EMBODIMENTS

The following detailed description contains, for the purposes of explanation, numerous specific embodiments, implementations, examples and details in order to provide a thorough understanding of the invention. It is apparent, however, that the embodiments may be practiced without these specific details or with an equivalent arrangement. In other instances, some well-known structures and devices are shown in block diagram form in order to avoid unnecessarily obscuring the embodiments of the invention. The description should in no way be limited to the illustrative implementations, drawings, and techniques illustrated below, including the exemplary designs and implementations illustrated and described herein, but may be modified within the scope of the appended claims along with their full scope of equivalents.

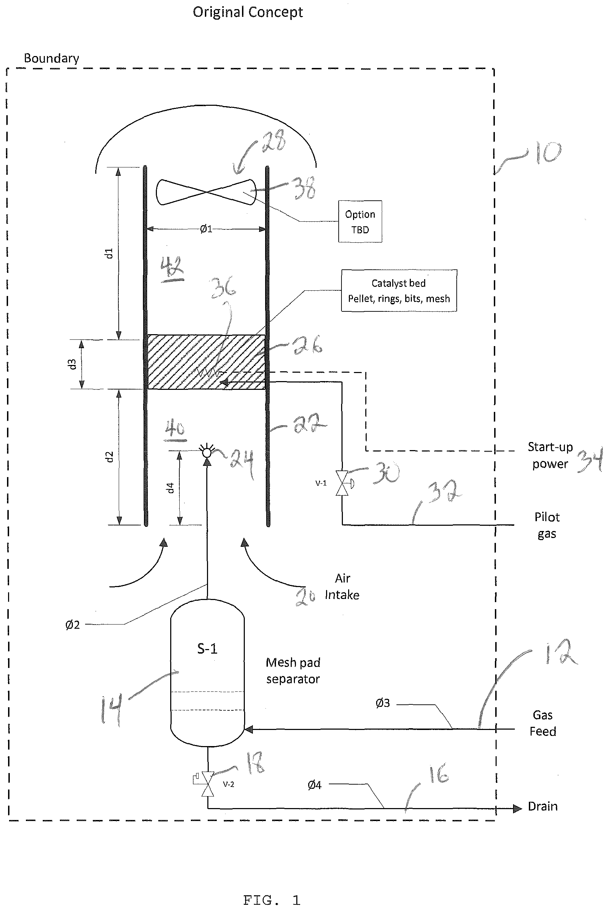

In a first embodiment of the present invention which is illustrated by way of example in FIG. 1, a methane converter 10 includes a gas feed 12 for feeding methane into the converter. In this embodiment, the converter also includes a mesh pad separator 14 for receiving the methane from the gas feed and for separating methane gas from liquid. In this embodiment, the converter includes a drain 16 connected to the separator to drain off the liquid. An optional valve 18 may be provided between the separator and the drain. The converter includes an air intake 20 for receiving air and a tubular section 22 extending upwardly from the air intake. The converter in this embodiment has a methane nozzle 24 connected to the separator for receiving the methane gas from the separator and for discharging the methane gas inside the tubular section at a point downstream of the air intake. As shown in FIG. 1, the converter has a catalyst bed 26 within the tubular section for reacting the methane gas with oxygen in the air to form the carbon dioxide and the water. The converter further includes an outlet 28 of the tubular section for emitting the carbon dioxide and the water. The converter of FIG. 1 thus acts as a chemical reactor to completely (or at least to very substantial degree) convert methane into carbon dioxide and water. Methane is considered to be 84% more potent than carbon dioxide as a greenhouse gas, thus making carbon dioxide a more environmentally acceptable gas to be released into the atmosphere. It will be appreciated that the embodiments of this invention may optionally be used in conjunction with any suitable carbon capture device to lessen the amount of carbon dioxide.

The catalyst bed may be a deep conversion catalyst bed, e.g. a packed bed membrane reactor or any other suitable catalytic converter or reactor.

In order to achieve complete conversion of methane to carbon dioxide and water it is preferable to employ an excess of oxygen to be combined with the methane. Since the reaction of methane and oxygen is endothermic, energy is required to initiate the reaction. This energy can be provided from a heat source, e.g. stack heat, waste heat, etc.

For the purposes of the present specification, the term "tubular" is used to describe a preferred shape or profile of the converter although it will be appreciated that the term shall be interpreted broadly to encompass any passageway having a hollow or partially hollow center portion surrounded by a solid perimeter. The term "tubular" should not be limited to only cylindrical shapes. It will also be appreciated that various other shapes or geometries may be used to implement the converter without departing from the inventive concept.

In the embodiment of FIG. 1, the converter may include a pilot gas valve 30 and a pilot line 32 for flowing a small quantity of the methane gas into the catalyst bed. The converter may also include an electrically powered start-up heat source having an electric power source 34 and a resistive heating element 36 for starting up the catalyst bed when gas is supplied to the catalyst bed.

The converter may use natural draft forces, e.g. in a stack. Alternatively, the converter may comprise a fan 38. The optional fan may be disposed at or near the outlet as illustrated.

In the first embodiment, the tubular section comprises an inlet section 40 between the intake and the catalyst bed. The tubular section further comprises an outlet section 42 between the catalyst bed and the outlet. In the particular configuration presented in FIG. 1, the inlet section and the outlet section are each longer than the catalyst bed. In the first embodiment, the inlet section, catalyst bed and outlet section have the same diameter.

The converter of FIG. 1 operates passively, i.e. without active mechanical switching. Furthermore, the converter of FIG. 1 is able to completely convert methane. Once it has been started up, the converter requires no continuous regulation, actuation or mechanical switching to operate. The converter may operate intermittently or continuously. Due to its robust nature, the converter may be used in hazardous locations.

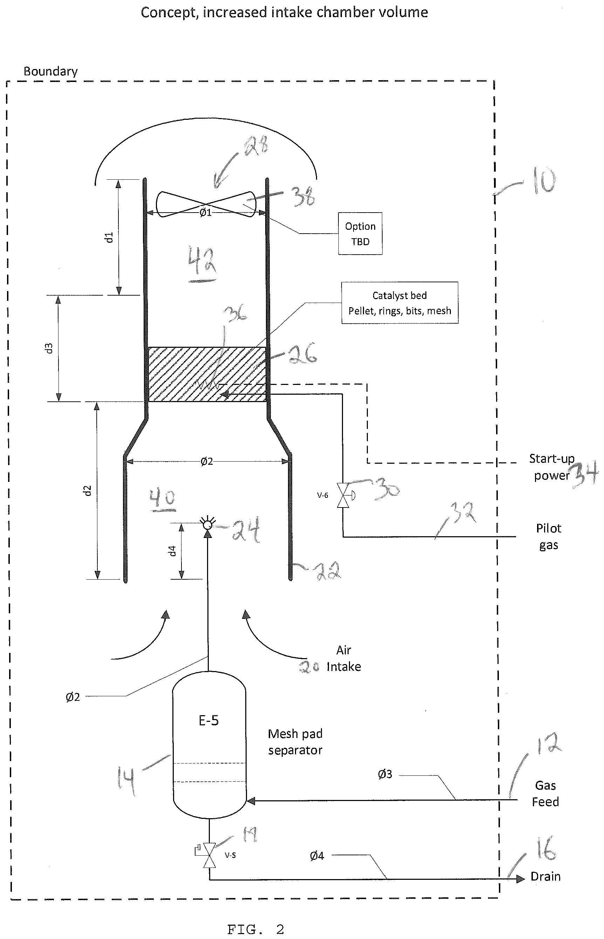

In a second embodiment of the present invention which is illustrated by way of example in FIG. 2, the methane converter has an air intake that is wider than the outlet to increase a volume of an inlet section (i.e. intake chamber).

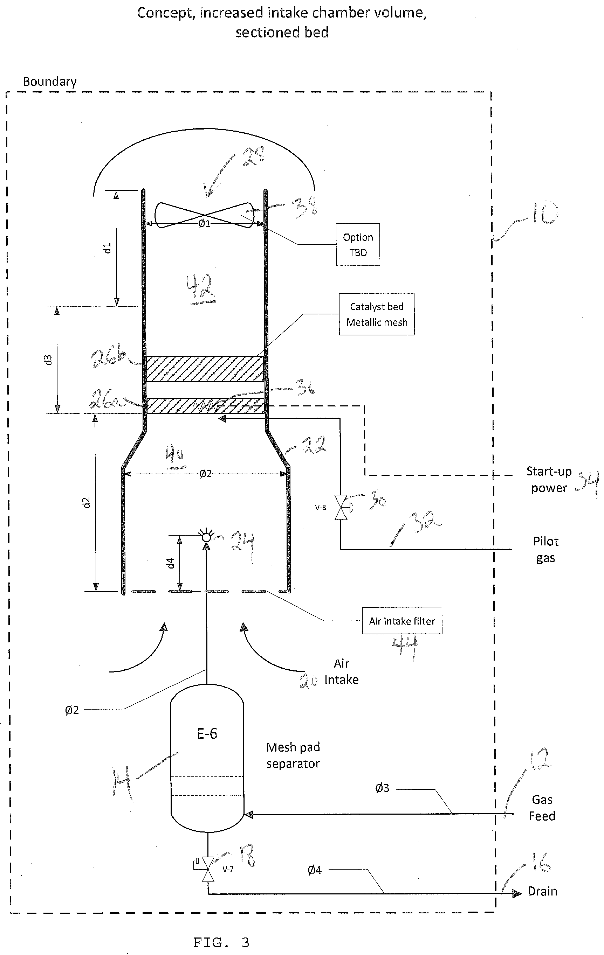

In a third embodiment of the present invention which is illustrated by way of example in FIG. 3, the methane converter has a first bed section 26a and a second bed section 26b. The second bed section is spaced downstream from the first bed section. In other words, between the first and second bed sections is a zone devoid of catalyst. In this third embodiment shown in FIG. 3, the methane nozzle is situated upstream of both the first and second bed sections. In this embodiment, the pilot gas valve delivers (via a pilot line) the small quantity of the methane gas to only the first bed section and the heat source is disposed only within the first bed section. In this embodiment, the second bed section neither has a heat source nor a pilot line.

In the embodiment of FIG. 3, the converter has an air intake filter 44. The air intake filter may be disposed at or near the air intake as illustrated.

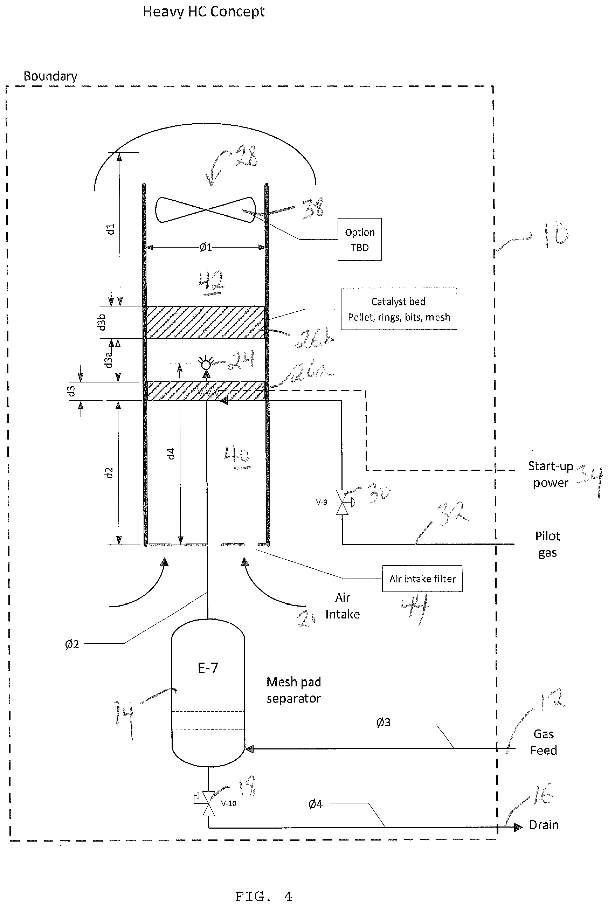

In a fourth embodiment of the present invention which is illustrated by way of example in FIG. 4, the methane nozzle is disposed between the first bed section and the second bed section. In other words, as illustrated in FIG. 4, the methane nozzle is situated downstream of the first bed section and upstream of the second bed section.

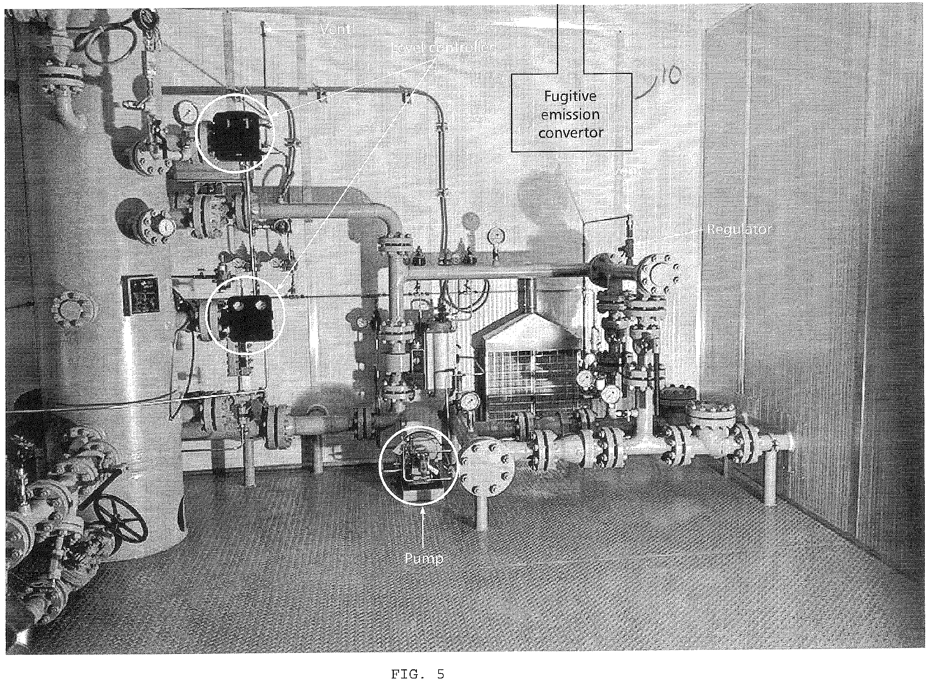

FIG. 5 depicts one exemplary use of the methane converter inside an oil facility. By using the methane converter in oil and gas facilities, the methane converter is able to convert fugitive and/or vented gases into less pernicious carbon dioxide. In one exemplary use, the methane converter may be disposed to receive fugitive methane emissions from oil and gas equipment. In another exemplary use, the methane converter may receive vented methane emissions for conversion. In addition to use in the oil and gas industries, the converter may be used in other plants or industries where methane is released such as power generation plants or steel mills.

It is to be understood that the singular forms "a", "an" and "the" include plural referents unless the context clearly dictates otherwise. Thus, for example, reference to "a device" includes reference to one or more of such devices, i.e. that there is at least one device. The terms "comprising", "having", "including", "entailing" and "containing", or verb tense variants thereof, are to be construed as open-ended terms (i.e., meaning "including, but not limited to,") unless otherwise noted. All methods described herein can be performed in any suitable order unless otherwise indicated herein or otherwise clearly contradicted by context. The use of examples or exemplary language (e.g. "such as") is intended merely to better illustrate or describe embodiments of the invention and is not intended to limit the scope of the invention unless otherwise claimed.

While several embodiments have been provided in the present disclosure, it should be understood that the disclosed systems and methods might be embodied in many other specific forms without departing from the scope of the present disclosure. The present examples are to be considered as illustrative and not restrictive, and the intention is not to be limited to the details given herein. For example, the various elements or components may be combined or integrated in another system or certain features may be omitted, or not implemented.

In addition, techniques, systems, subsystems, and methods described and illustrated in the various embodiments as discrete or separate may be combined or integrated with other systems, modules, techniques, or methods without departing from the scope of the present disclosure. Other items shown or discussed as coupled or directly coupled or communicating with each other may be indirectly coupled or communicating through some interface, device, or intermediate component whether electrically, mechanically, or otherwise. Other examples of changes, substitutions, and alterations are ascertainable by one skilled in the art and could be made without departing from the inventive concept(s) disclosed herein.

## Claims

1. A methane converter for converting methane into carbon dioxide and water, the converter comprising:
a gas feed for feeding methane into the converter;
a mesh pad separator for receiving the methane from the gas feed and for separating methane gas from liquid;
a drain connected to the separator to drain off the liquid;
an air intake for receiving air;
a tubular section extending upwardly from the air intake;
a methane nozzle connected to the separator for receiving the methane gas from the separator and for discharging the methane gas inside the tubular section at a point downstream of the air intake;
a catalyst bed within the tubular section for reacting the methane gas with oxygen in the air to form the carbon dioxide and the water; and
an outlet of the tubular section for emitting the carbon dioxide and the water.

2. The converter of claim 1 comprising a pilot gas valve for flowing a small quantity of the methane gas into the catalyst bed and an electrically powered start-up heat source for starting up the catalyst bed.

3. The converter of claim 1 further comprising a fan at or near the outlet.

4. The converter of claim 1 wherein the tubular section comprises an inlet section between the intake and the catalyst bed and wherein the tubular section further comprises an outlet section between the catalyst bed and the outlet, wherein the inlet section and the outlet section are each longer than the catalyst bed.

5. The converter of claim 4 wherein the inlet section, catalyst bed and outlet section have the same diameter.

6. The converter of claim 1 wherein the air intake is wider than the outlet.

7. The converter of claim 1 wherein the catalyst bed comprises a first bed section and a second bed section spaced downstream from the first bed section.

8. The converter of claim 7 wherein the pilot gas valve delivers the small quantity of the methane gas to only the first bed section and wherein the heat source is disposed only within the first bed section.

9. The converter of claim 7 wherein the methane nozzle is situated upstream of both the first and second bed sections.

10. The converter of claim 7 wherein the methane nozzle is situated downstream of the first bed section and upstream of the second bed section.

11. The converter of claim 7 further comprising an air filter at or near the air intake.

12. A method of converting methane into carbon dioxide and water, the method comprising:
feeding methane into the converter via a gas feed;
separating methane gas from liquid using a mesh pad separator;
draining off the liquid from the separator;
receiving air into the converter via an air intake;
flowing the air upwardly from the air intake through a tubular section;
discharging the methane gas received from the separator through a methane nozzle inside the tubular section at a point downstream of the air intake;
reacting, in a catalyst bed within the tubular section, the methane gas with oxygen in the air to form the carbon dioxide and the water; and
emitting the carbon dioxide and the water via an outlet of the tubular section.

13. The method of claim 12 further comprising:
flowing a small quantity of the methane gas into the catalyst bed using a pilot gas valve; and
starting up the catalyst bed using an electrically powered start-up heat source.

14. The method of claim 13 wherein reacting is performed using a first bed section and a second bed section spaced downstream from the first bed section and wherein flowing the small quantity of the methane gas into the catalyst bed comprises delivering the small quantity of the methane gas to only the first bed section and wherein the heat source is disposed only within the first bed section.

15. The method of claim 14 wherein discharging the methane gas is performed by situating the nozzle upstream of both the first and second bed sections wherein discharging the methane gas is performed by situating the nozzle downstream of the first bed section and upstream of the second bed section.
